# EUROPEAN PATENT SPECIFICATION

(11) **EP 2 498 874 B1**
(45) Date of publication and mention of the grant of the patent: **20.09.2017**
(21) Application number: 10830593.9
(22) Date of filing: 09.11.2010
(51) Int. Cl.: A61N 1/378, A61N 1/36

(54) **IMPLANT POWER SYSTEM**
IMPLANTATLEISTUNGSSYSTEM
SYSTÈME D'ALIMENTATION D'IMPLANT

(30) Priority: 10.11.2009 US 259854 P
(43) Date of publication of application: 19.09.2012
(73) Proprietor: MED-EL Elektromedizinische Geräte GmbH, 6020 Innsbruck (AT)
(72) Inventor: ZIERHOFER, Clemens, M., A-6250 Kundl (AT)
(74) Representative: Lucke, Andreas
(86) International application number: PCT/US2010/056009
(87) International publication number: WO 2011/059971

(56) References cited:
- WO-A1-99/07302
- US-A1- 2002 099 421
- US-A1- 2004 073 275
- US-A1- 2005 075 694
- US-A1- 2005 075 696
- US-A1- 2005 075 696
- US-A1- 2006 184 213
- US-A1- 2007 150 009
- US-B1- 6 275 737
- US-B1- 6 308 101

## Description

### Technical Field

The present invention relates to implants, and more particularly, to a system and methodology to recharge a battery in an external power piece associated with a cochlear implant.

### Background Art

Cochlear implants and other inner ear prostheses are one option to help profoundly deaf or severely hearing impaired persons. Unlike conventional hearing aids that just apply an amplified and modified sound signal; a cochlear implant is based on direct electrical stimulation of the acoustic nerve. Typically, a cochlear implant stimulates neural structures in the inner ear electrically in such a way that hearing impressions most similar to normal hearing is obtained.

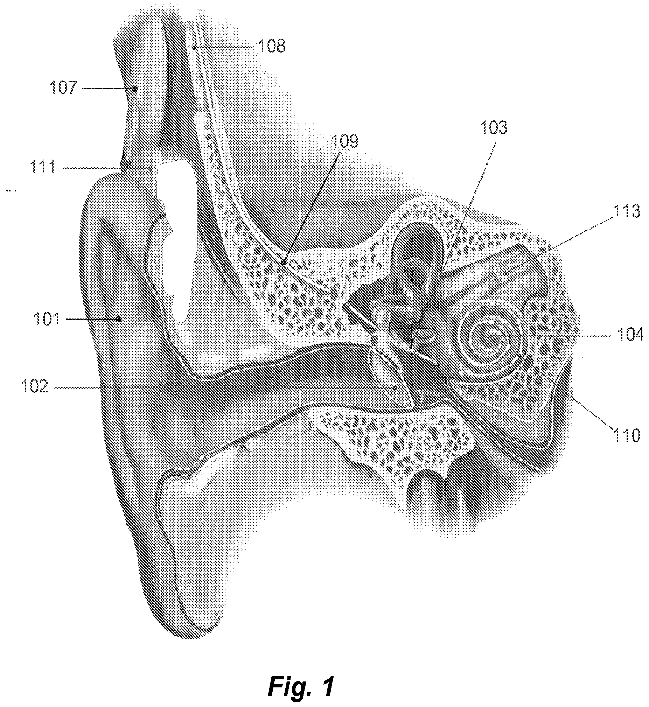

More particularly, a normal ear transmits sounds as shown in Figure 1 through the outer ear 101 to the tympanic membrane (eardrum) 102, which moves the bones of the middle ear 103 (malleus, incus, and stapes) that vibrate the oval window and round window openings of the cochlea 104. The cochlea 104 is a long narrow duct wound spirally about its axis for approximately two and a half turns. It includes an upper channel known as the scala vestibuli and a lower channel known as the scala tympani, which are connected by the cochlear duct. The cochlea 104 forms an upright spiraling cone with a center called the modiolar where the spiral ganglion cells of the acoustic nerve 113 reside. In response to received sounds transmitted by the middle ear 103, the fluid-filled cochlea 104 functions as a transducer to generate electric pulses which are transmitted to the cochlear nerve 113, and ultimately to the brain.

Some persons have partial or full loss of normal sensorineural hearing. Cochlear implant systems have been developed to overcome this by directly stimulating the user's cochlea 104. One type of cochlear prosthesis may include two parts: a speech processor 111 and the implanted stimulator 108. The speech processor 111 typically includes a power supply (batteries) for the overall system, a microphone, and a processor that is used to perform signal processing of the acoustic signal to extract the stimulation parameters. The speech processor may be a behind-the-ear (BTE-) device.

The stimulator 108 generates the stimulation patterns (based on the extracted audio information) that are sent through an electrode lead 109 to an implanted electrode array 110. Typically, this electrode array 110 includes multiple electrodes on its surface that provide selective stimulation of the cochlea 104. For example, each electrode of the cochlear implant is often stimulated with signals within an assigned frequency band based on the organization of the inner ear. The placement of each electrode within the cochlea is typically based on its assigned frequency band, with electrodes closer to the base of the cochlea generally corresponding to higher frequency bands.

The connection between the speech processor and the stimulator is usually established by means of a radio frequency (RF-) link, which may utilize coils for the transcutaneous transmission of RF/power signals. Note that via the RF-link both stimulation energy and stimulation information are conveyed. Typically, digital data transfer protocols employing bit rates of some hundreds of kBit/s are used.

A totally implantable cochlear implant (TICI) is a cochlear implant system without permanently used external components such as an external speech processor. The implantable TICI typically includes a microphone and subsequent stages perform audio signal processing for the implementation of a particular stimulation strategy (e.g., CIS). It also includes stimulation electrodes, power management electronics, and a coil for the transcutaneous transmission of RF signals.

Unlike a pacemaker implant, the power supply of a TICI generally cannot be established by means of a non-rechargeable battery. This is because the overall pulse repetition rate of a cochlear implant is much higher. For example, typically about 20kpulses/s are generated by a cochlear implant using CIS, as compared to about 1pulse/s in a pacemaker. Besides, a cochlear implant typically performs complex audio signal processing, as compared to simple sensing tasks performed in a pacemaker. Consequently, a rechargeable battery is typically required in a cochlear implant, which needs recharging after a particular time period of operation. To recharge the battery of a TICI, an External Power Supply Module (EPSM) is used for transcutaneous transmission of RF/power signals.

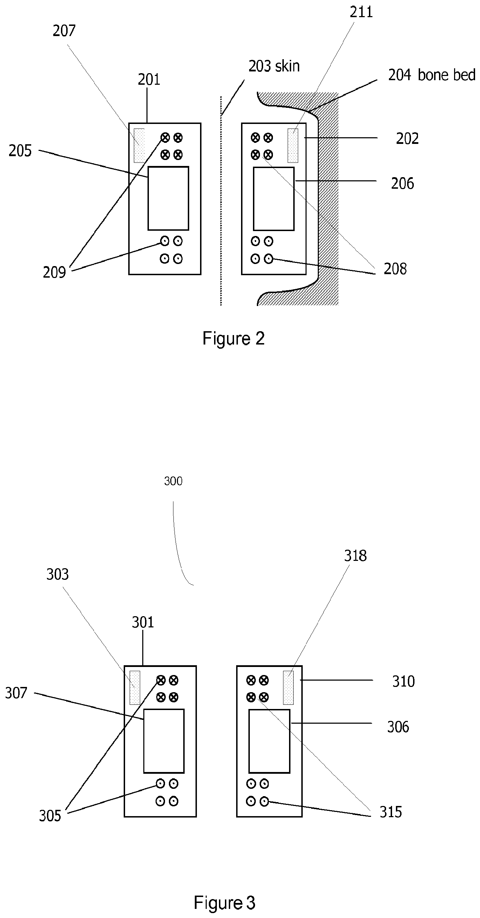

As shown in Fig. 2, the typical EPSM 201 may include includes a first magnet 205, a rechargeable battery 207, and a coil 209 for transcutaneous transmission of a power signal. A TICI 202 located under the skin 203 and embedded in bone 204 typically includes a second magnet 206, a coil 208 for receiving the power signal, and a rechargeable battery 211 that is recharged with the received power signal. The first magnet 205 is positioned over the second magnet 206 such that the EPSM 201 is held against the implant 202 in an optimum position. By maintaining such a position, the external coil 209 associated with EPSM 201 can, via inductive coupling, transmit power to the coil 208 of implant 202.

To recharge the battery of the EPSM 201, the EPSM 201 typically must be opened to remove the rechargeable battery 207. The rechargeable battery 207 is then placed in a battery recharger. This process may be inconvenient and/or confusing, especially for younger children. Additionally, there is the risk that the battery 207 may be misplaced or otherwise separated from the TICI. Requiring that the EPSM 201 be opened multiple times for battery removal also complicates efforts to make the EPSM 201 waterproof.

US 20070150009 discloses a pacing apparatus system having a stent electrode through which pulses of electrical current can be delivered. In one embodiment, the stent electrode is powered using an implanted pacing controller housed in a single body implanted enclosure together with a transmitter and a rechargeable battery. The transmitter is further connected with a loop antenna which provides supply power and information signals to the stent electrode. Further, the loop antenna can be used for recharging the implanted battery by receiving RF energy from a source outside the body.

In an alternative embodiment, only the pacing controller is implantable, whereas the transmitter along with the loop antenna is adapted to be worn externally, such as in a harness that is worn by the patient. In this case, the implanted pacing controller has its own energy source, such as an electrochemical battery, which is not rechargeable, given the relatively small energy requirements of a pacing controller as such.

US 20040073275 discloses a cochlear implant sound processor which is powered by a rechargeable battery that is permanently integrated into the sound processor. The sound processor contains an inductive coil that may be tuned to an external charging coil for battery recharging. The electronic circuits and coil of the sound processor are housed in a material transparent to RF signals. The sound processor may be placed in a recharging base station in which the sound processor is positioned in a space surrounded by the inductive charging coil embedded in a material transparent to RF signals. The inductive charging coil sends power to the coil inside the speech processor and thereby recharges the battery. The sound processor is further coupled with a head piece via a cable. The head piece includes a further coil for inductive or RF-coupling with a coil in a cochlear implant. When recharging the sound processor, the additional coil of the head piece is coupled with a yet further coil in the recharging base, allowing the speech processor communicating with the charging base for carrying out diagnostic routines and the like.

A similar charger system for implantable medical devices is also disclosed in US 2005/0075696.

### Summary of the Invention

In accordance with the invention, a power supply system for a cochlear implant as defined in claim 1 is provided.

In accordance with another aspect of the invention, a method of recharging a battery of an external power supply module as defined in claim 7 is presented. Preferred embodiments are defined in the dependent claims. Embodiments, aspects and examples disclosed herein, but not falling under the scope of claim 1 or 7, do not form part of the invention.

### Brief Description of the Drawings

The foregoing features of the invention will be more readily understood by reference to the following detailed description, taken with reference to the accompanying drawings, in which:
Fig. 1 illustrates a sectional view of an ear connected to a cochlear implant system (PRIOR ART);
Fig. 2 is a graphical illustration of a Totally Implantable Cochlear Implant (TICI) and external part used for transfer of power (PRIOR ART);
Fig. 3 is a graphical illustration of an external power supply module and an external charger, in accordance with an embodiment of the invention;.
Fig.4 shows an illustrative circuit of an external power supply module (EPSM) and an associated implant, in accordance to an embodiment of the present invention; and
Fig. 5 shows an illustrative circuit of an external power supply module (EPSM) including a rechargeable battery that includes a Greinacher circuit, in accordance to an embodiment of the present invention.

### Detailed Description of Specific Embodiments

In illustrative embodiments, a system and method for recharging a battery of an external power supply module (EPSM) associated with an implant is presented. The EPSM includes a first coil for transmitting power across the skin of a user. An external charger has a second coil that transfers power to the first coil of the EPSM, without having to open the EPSM. Various embodiments of the EPSM are advantageously made waterproof. Further details are described below.

Fig. 3 is a graphical illustration of a power system 300 that includes an EPSM 301 and an external charger 310, in accordance with an embodiment of the invention. The EPSM module 301 includes a first power signal transmission module. The first power signal transmission module includes a first rechargeable battery 303 and a first coil 305 for transcutaneously transmitting power from the battery 303 to any one of a variety of implants (not shown), such as a cochlear implant, a defibrillator, a cardioverter, a pacemaker, and a retinal implant. The cochlear implant may be a TICI that includes a speech processor. Alternatively, the speech processor may be located externally, such as in combination with the EPSM. In such embodiments, both power and data may be transmitted from the first coil 305 to the implant.

In various embodiments, the EPSM 301 may include a first magnet 307 that is positioned over a second magnet of the implant, such that the EPSM 301 is held against the implant in an optimum position, as described above with regard to a TICI. By maintaining such a position, the first coil 305 associated with the EPSM 301 can, via inductive coupling, transmit power to, without limitation, a third coil associated with the implant. More particularly, the first coil 305 generates a field that is picked up and converted into electric current by the third coil within the implant when it is aligned with said first coil 305. The power received by the implant may be used, for example, to provide power to electronic circuitry within the implant and/or to recharge a battery within the implant.

The external charger 310 is advantageously capable of recharging the first battery 303 without opening the EPSM 301. For example, the charger 310 may include a second power signal transmission module having a second coil 315 for transmitting a second electrical power signal to the first coil 305. By placing the EPSM 301 in an optimum position with regard to the charger 310, the charger 310 can, via inductive coupling, transmit power from the second coil 315 to the first coil 305 of the EPSM 301.

To recharge the battery of the EPSM 301, the user may place the EPSM 301 into a docking base of the charger 310. In various embodiments, the housings of the charger 310 and the EPSM 301 may be shaped such that the EPSM 301 is guided into the optimum position for transmission of power. The charger may also include a magnet 306 that works with magnet 307 of the EPSM 301 to optimally position the EPSM 301. Typically, the best performance is obtained when the two coils 305 and 315 of the EPSM 310 and charger 310, respectively, are perfectly aligned. The housing of the EPSM 301 may include, for example, teeth that mate with corresponding slots in the base of charger 310 to align the two housing portions, or various guide rails.

Various feedback may be provided to the user when the EPSM 301 is positioned in the optimum position relative to the charger 310. For example, the EPSM 301 or charger 310 may provide, without limitation, an indicator light and/or clicking sound when in the optimum position. Indications of correct operation, power transfer, voltage/current levels, and other status may also be provided in the charger 310 and/or EPSM 301.

The charger 310 may be powered by connecting to wall socket and using conventional house current. In other embodiments, the charger 310 may include its own internal battery 318, which may be rechargeable by, without limitation, plugging into an available power source. Using rechargeable batteries within the charger 310 may be convenient when a power source is not available, for example, when in an outside environment, such as camping.

In illustrative embodiments, the EPSM 301 includes a waterproof housing. This capability is more easily incorporated into the EPSM 301 of the current invention, as the user does not have open the EPSM 301 to recharge the battery 303. The housing of the EPSM 301 may be held together by, without limitation, a sealant or a weld seam. For example, ultrasonic welding or a UV curable sealant may be used.

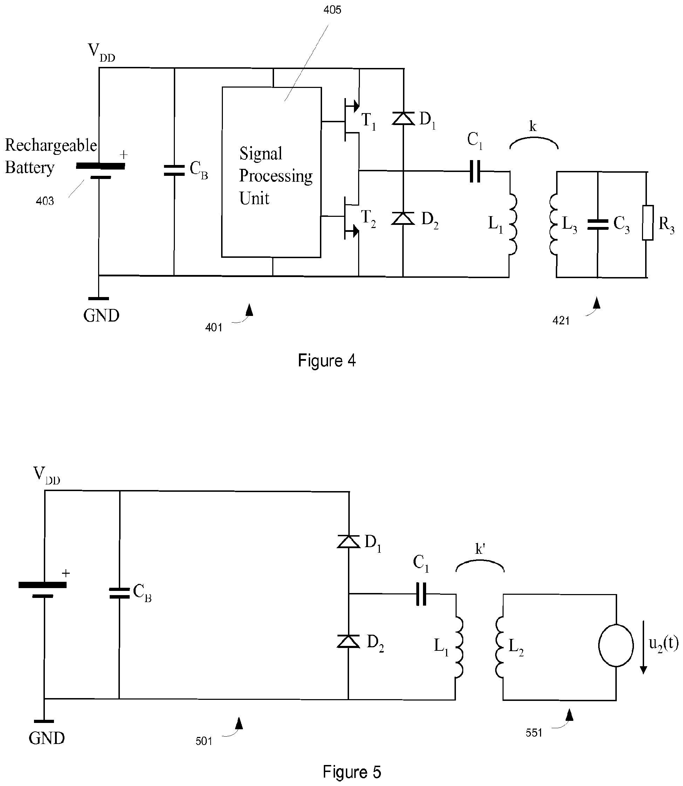

Fig.4 shows an illustrative circuit of an EPSM 401 and an associated implant 421, in accordance to an embodiment of the present invention. The EPSM 401 advantageously includes a class-D switching scheme for transmitting, without limitation, power or data, that can be integrated onto a single microchip. Thus, power consumption and system size may be kept very small. Also, a signal processing unit 405 for driving switches T1 and T2 can easily be integrated onto such a microchip. More particularly, embodiment of the EPSM 401 may include a rechargeable battery 403, a stabilization capacitor CB, a signal processing unit 405, class-D switching transistors T1 and T2, protection diodes D1 and D2, and a series tuned RF-circuit C1, L1. The EPSM is inductively coupled to an implant (coupling factor k) 421, which is here illustratively represented by a parallel tuned RF-circuit L3, C3, and R3.

Fig. 5 shows an illustrative circuit of an EPSM 501 including a rechargeable battery 503 along with an external charger 551, in accordance to an embodiment of the present invention. The external charger 551 is illustratively represented by coil L2 driven by the RF-voltage source u2(t). Coils L2 and EPSM-coil L1 are inductively coupled (coupling factor k'). Network C1, D1, D2, CB represent the basic topology of a so called "Greinacher circuit". The Greinacher circuit is a textbook approach for voltage doubling.

Although various exemplary embodiments of the invention have been disclosed, it should be apparent to those skilled in the art that various changes and modifications can be made that will achieve some of the advantages of the invention without departing from the true scope of the invention as defined by the appended claims.

## Claims

1. A power supply system (300) for a cochlear implant, the system comprising:
an external power supply module (301) including:
- a first power signal transmission module including a first coil (305) for transmitting a first electrical power signal across the skin of a user to the cochlear implant; and
- a first rechargeable battery (303) for supplying power to the first power signal transmission module;
and an external charger (310) for recharging the first rechargeable battery (303); the charger including a second power signal transmission module including a second coil (315) for transmitting a second electrical power signal to the first coil (305),
wherein said power supply system (300) further comprises the cochlear implant, the cochlear implant including a third coil for receiving the first electrical power signal,
when implanted, for providing power to electronic circuitry within the cochlear implant and/or charging a battery within the cochlear implant, if present.

2. The power supply system (300) according to claim 1, wherein the cochlear implant further includes a second rechargeable battery for supplying power to the cochlear implant, and wherein the second rechargeable battery is recharged within the cochlear implant using the first electrical power signal.

3. The power supply system according to claim 1, wherein the cochlear implant includes a speech processor, or
wherein the external power supply module (301) includes a speech processor, and wherein the speech processor transmits data from the first coil (305) to the third coil.

4. The power supply system (300) according to claim 1, wherein the external power supply module (301) is enclosed in a waterproof housing.

5. The power supply system (300) according to claim 1, wherein the charger (310) is adapted to transmit the second electrical power signal from the second coil (315) to the first coil (305) without opening the waterproof housing.

6. The power supply system (300) according to claim 1, wherein the first power signal transmission module includes a class D switching module.

7. A method of transferring power in a cochlear implant system, the system including an external power supply module (301), the external power supply module (301) including a first power signal transmission module including a first coil (305) for transmitting a first electrical power signal across the skin of a user to a cochlear implant, the external power supply module (301) further including a first rechargeable battery (303) for supplying power to the first power signal transmission module, the method comprising:
transmitting a second electrical power signal from a second coil (315) to the first coil (305); and
recharging the first rechargeable battery (303) with the second electrical power signal.

8. The method according to claim 7, wherein said cochlear implant system further comprises a cochlear implant, the cochlear implant including a third coil and a second rechargeable battery, the second rechargeable battery for supplying power to the implant, the method further comprising:
receiving the first electrical power signal at the third coil;
recharging the second rechargeable battery with the first electrical power signal.

9. The method according to claim 7,
wherein the cochlear implant includes a speech processor.

10. The method according to claim 7, wherein the external power supply module (301) includes a speech processor for providing stimulation data, the method further comprising transmitting the stimulation data from the first coil (305) to a third coil.

11. The method according to claim 7, further comprising enclosing the external power supply module (301) in a waterproof housing.

12. The method according to claim 11, wherein transmitting the second electrical power signal and recharging the first rechargeable battery (305) is performed without opening the waterproof housing.

13. The method of claim 7, wherein transmitting the second electrical power signal includes positioning the second coil (315) so that it is aligned with the first coil.

14. The method according to claim 7, wherein the external power supply module (301) includes a class D switching module, and wherein the method includes using the class D switching module to form the first electrical power signal.

## Patentansprüche

1. Leistungsversorgungssystem (300) für ein Cochleaimplantat, wobei das System folgendes umfasst:
ein externes Leistungsversorgungsmodul (301), das folgendes umfasst:
- ein erstes Leistungssignal-Übertragungsmodul, welches eine erste Spule (305) zum Übertragen eines ersten elektrischen Leistungssignals durch die Haut eines Nutzers zu dem Cochleaimplantat umfasst; und
- eine erste wiederaufladbare Batterie (303), um das erste Leistungssignal-Übertragungsmodul mit Leistung zu versorgen;
und ein externes Ladegerät (310) zum Wiederaufladen der ersten wiederaufladbaren Batterie (303); wobei das Ladegerät ein zweites Leistungssignal-Übertragungsmodul umfasst,
welches eine zweite Spule (315) enthält, um ein zweites elektrisches Leitungssignal zu der ersten Spule (305) zu übertragen,
wobei das Leistungsversorungssystem (300) ferner das Cochleaimplantat umfasst, wobei das Cochleaimplantat eine dritte Spule zum Empfangen des ersten elektrischen Leistungssignals enthält, wenn es implantiert ist, um elektrische Schaltungen innerhalb des Cochleaimplantats mit Leistung zu versorgen und/oder eine Batterie in dem Cochleaimplantat aufzuladen, falls eine solche vorhanden ist.

2. Leistungsversorgungssystem (300) nach Anspruch 1, bei dem das Cochleaimplantat ferner eine zweite wiederaufladbare Batterie zum Versorgen des Cochleaimplantats mit Leistung umfasst, und wobei die zweite weideraufladbare Batterie innerhalb des Cochleaimplantats unter Verwendung des ersten elektrischen Leistungssignals wiederaufgeladen wird.

3. Leistungsversorgungssystem nach Anspruch 1, wobei das Cochleaimplantat einen Sprachprozessor umfasst, oder
wobei das externe Leistungsmodul (301) einen Sprachprozessor umfasst, und wobei der Sprachprozessor Daten von der ersten Spule (305) zu der dritten Spule überträgt.

4. Leistungsversorgungssystem (300) nach Anspruch 1, wobei das externe Leistungsversorgungsmodul (301) in einem wasserdichten Gehäuse eingeschlossen ist.

5. Leistungsversorgungssystem (300) nach Anspruch 1, wobei das Ladegerät (310) geeignet ist, das zweite elektrische Leistungssignal von der zweiten Spule (315) zu der ersten Spule (305) zu übertragen, ohne das wasserdichte Gehäuse zu öffnen.

6. Leistungsversorgungssystem (300) nach Anspruch 1, wobei das erste Leistungssignal-Übertragungsmodul ein Klasse-D-Schaltmodul umfasst.

7. Verfahren zum Übertragen von Leistung in einem Cochleaimplantatsystem, wobei das System ein externes Leistungsversorgungsmodul (301) umfasst, wobei das externe Leistungsversorgungsmodul (301) ein erstes Leistungssignal-Übertragungsmodul umfasst, welches eine erste Spule (305) zum Übertragen eines ersten elektrischen Leistungssignals durch die Haut eines Nutzers zu einem Cochleaimplantat enthält, wobei das externe Leistungsversorgungsmodul (301) ferner eine erste wiederaufladbare Batterie (303) umfasst, um das erste Leistungssignal-Übertragungsmodul mit Leistung zu versorgen, wobei das Verfahren folgendes umfasst:
Übertragen eines zweiten elektrischen Leistungssignals von einer zweiten Spule (315) zu der ersten Spule (305);
Wiederaufladen der ersten wiederaufladbaren Batterie (303) mit dem zweiten elektrischen Leistungssignal.

8. Verfahren nach Anspruch 7, wobei das Cochleaimplantatsystem ferner ein Cochleaimplantat umfasst, wobei das Cochleaimplantat eine dritte Spule und eine zweite wiederaufladbare Batterie umfasst, wobei die zweite wiederaufladbare Batterie dazu bestimmt ist, das Implantat mit Leistung zu versorgen, wobei das Verfahren ferner folgendes umfasst:
Empfangen des ersten elektrischen Leistungssignals an der dritten Spule;
Wiederaufladen der zweiten wiederaufladbaren Batterie mit dem ersten elektrischen Leistungssignal.

9. Verfahren nach Anspruch 7,
wobei das Cochleaimplantat einen Sprachprozessor umfasst.

10. Verfahren nach Anspruch 7, bei dem das externe Leistungsversorgungsmodul (301) einen Sprachprozessor zum Bereitstellen von Stimulationsdaten umfasst, wobei das Verfahren ferner das Übertragen der Stimulationsdaten von der ersten Spule (305) zu einer dritten Spule umfasst.

11. Verfahren nach Anspruch 7, das ferner das Einschließen des externen Leistungsversorgungsmoduls (301) in einem wasserdichten Gehäuse umfasst.

12. Verfahren nach Anspruch 11, wobei das Übertragen des zweiten elektrischen Leistungssignals und das Wiederaufladen der ersten wiederaufladbaren Batterie (305) durchgeführt werden, ohne das wasserdichte Gehäuse zu öffnen.

13. Verfahren nach Anspruch 7, wobei das Übertragen des zweiten elektrischen Leistungssignals das Positionieren der zweiten Spule (315) derart umfasst, dass sie mit der ersten Spule ausgerichtet ist.

14. Verfahren nach Anspruch 7, wobei das externe Leistungsversorgungsmodul (301) ein Klasse-D-Schaltmodul umfasst, und wobei das Verfahren ferner die Verwendung des Klasse-D-Schaltmoduls zum Bilden des ersten elektrischen Leistungssignals umfasst.

## Revendications

1. Système d'alimentation électrique (300) destiné à un implant cochléaire, le système comprenant :
un module d'alimentation électrique externe (301) comprenant :
- un premier module de transmission de signal d'alimentation qui comprend une première bobine (305) destinée à transmettre un premier signal d'alimentation électrique à travers la peau d'un utilisateur, jusqu'à l'implant cochléaire ; et
- une première batterie rechargeable (303) destinée à fournir de l'énergie au premier module de transmission de signal d'alimentation ;
et un chargeur externe (310) destiné à recharger la première batterie rechargeable (303) ; le chargeur comprenant un second module de transmission de signal d'alimentation qui comprend une seconde bobine (315) destinée à transmettre le second signal d'alimentation électrique à la première bobine (305),
dans lequel ledit système d'alimentation électrique (300) comprend en outre l'implant cochléaire, l'implant cochléaire comprenant une troisième bobine destinée à recevoir le premier signal d'alimentation électrique, lorsqu'il est implanté, afin de fournir de l'énergie aux circuits électroniques situés à l'intérieur de l'implant cochléaire et/ou de recharger la batterie située à l'intérieur de l'implant cochléaire, le cas échéant.

2. Système d'alimentation électrique (300) selon la revendication 1, dans lequel l'implant cochléaire comprend en outre une seconde batterie rechargeable destinée à fournir de l'énergie à l'implant cochléaire, et dans lequel la seconde batterie rechargeable est rechargée à l'intérieur de l'implant cochléaire à l'aide du premier signal d'alimentation électrique.

3. Système d'alimentation électrique (300) selon la revendication 1, dans lequel l'implant cochléaire comprend un processeur vocal, ou
dans lequel le module d'alimentation électrique externe (301) comprend un processeur vocal, et
dans lequel le processeur vocal transmet des données entre la première bobine (305) et la troisième bobine.

4. Système d'alimentation électrique (300) selon la revendication 1, dans lequel le module d'alimentation électrique externe (301) est contenu dans un boîtier étanche à l'eau.

5. Système d'alimentation électrique (300) selon la revendication 1, dans lequel le chargeur (310) est adapté pour transmettre le second signal d'alimentation électrique entre la seconde bobine (315) et la première bobine (305) sans avoir à ouvrir le boîtier étanche à l'eau.

6. Système d'alimentation électrique (300) selon la revendication 1, dans lequel le premier module de transmission de signal d'alimentation comprend un module de commutation de classe D.

7. Procédé de transfert d'énergie dans un système d'implant cochléaire, le système comprenant un module d'alimentation externe (301), le module d'alimentation externe (301) comprenant un premier module de transmission de signal d'alimentation qui comprend une première bobine (305) destinée à transmettre un premier signal d'alimentation électrique à travers la peau d'un utilisateur jusqu'à un implant cochléaire, le module d'alimentation externe (301) comprenant en outre une première batterie rechargeable (303) destinée à fournir de l'énergie au premier module de transmission de signal d'alimentation, le procédé comprenant :
la transmission d'un second signal d'alimentation électrique entre une seconde bobine (315) et la première bobine (305) ; et
le rechargement de la première batterie rechargeable (303) à l'aide du second signal d'alimentation électrique.

8. Procédé selon la revendication 7, dans lequel le système d'implant cochléaire comprend en outre un implant cochléaire, l'implant cochléaire comprenant une troisième bobine et une seconde batterie rechargeable, la seconde batterie rechargeable étant destinée à fournir de l'énergie à l'implant, le procédé comprenant en outre :
la réception du premier signal d'alimentation électrique au niveau de la troisième bobine ;
le rechargement de la seconde batterie rechargeable avec le premier signal d'alimentation électrique.

9. Procédé selon la revendication 7, dans lequel l'implant cochléaire comprend un processeur vocal.

10. Procédé selon la revendication 7, dans lequel le module d'alimentation externe (301) comprend un processeur vocal destiné à fournir des données de stimulation, le procédé comprenant en outre la transmission des données de stimulation entre la première bobine (305) et la troisième bobine.

11. Procédé selon la revendication 7, comprenant en outre le placement du module d'alimentation externe (301) dans un boîtier étanche à l'eau.

12. Procédé selon la revendication 11, dans lequel la transmission du second signal d'alimentation électrique et le rechargement de la première batterie rechargeable (305) sont réalisés sans ouvrir le boîtier étanche à l'eau.

13. Procédé selon la revendication 7, dans lequel la transmission du second signal d'alimentation électrique comprend le positionnement de la seconde bobine (315) de façon à l'aligner avec la première bobine.

14. Procédé selon la revendication 7, dans lequel le module d'alimentation électrique externe (301) comprend un module de commutation de classe D, et dans lequel le procédé comprend l'utilisation du module de commutation de classe D afin de former le premier signal d'alimentation électrique.
